# EUROPEAN PATENT SPECIFICATION

(11) **EP 1 833 365 B1**
(45) Date of publication and mention of the grant of the patent: **13.02.2013**
(21) Application number: 04809195.3
(22) Date of filing: 23.12.2004
(51) Int. Cl.: A61B 5/08, A61N 1/37, A61B 5/053

(54) **MEDICAL DEVICE**
MEDIZINISCHE VORRICHTUNG
DISPOSITIF MEDICAL

(43) Date of publication of application: 19.09.2007
(73) Proprietor: St. Jude Medical AB, 175 84 Järfälla (SE)
(72) Inventor: HOLMSTRÖM, Nils, S-175 57 Järfälla (SE); ÖHLANDER, Malin, S-188 48 Stockholm (SE); KALLING, Sven, S-183 56 Täby (SE)
(74) Representative: Sjölander, Henrik
(86) International application number: PCT/SE2004/002022
(87) International publication number: WO 2006/068566

(56) References cited:
- US-A- 5 957 861
- US-A- 6 104 949
- US-A1- 2004 102 712
- US-B1- 6 473 640

## Description

### TECHNICAL FIELD

The present invention generally relates to implantable medical devices, such as cardiac pacemakers and implantable cardioverter/defibrillators, and in particular to an improved medical device for obtaining electrical bio-impedance signals in order to monitor or detect changes in a condition of the heart of a patient.

### BACKGROUND OF THE INVENTION

Today, in the modem society, heart diseases and/or conditions leading to an impaired heart function are a major problem entailing constantly increasing costs for medical services. For example, heart failure is a condition which affects thousands of people throughout the world. Congestive heart failure (CHF) is an inability of the heart to pump blood at an adequate rate in response to the filling pressure. Patients suffering from CHF are often afflicted by cardogenic pulmonary edema, which is caused by the accumulation of fluid in the lung interstitium and alveoli due to the fact the left ventricular venous return exceeds left ventricular cardiac output. That is, more fluids are transported to the lung region than from the lung region causing the accumulation of fluids in the lung region. CHF may even, in its more severe stages, result in death.

The progression of fluid accumulation in pulmonary edema, whether it is initiated by damage to the various components of the alveolar-capillary membranes or is cardiogenic in nature, can be identified by three distinct physiological stages. Stage I: Fluid and colloid shift into the lung interstitium from pulmonary capillaries, but an increase in lymphatic outflow efficiently removes the fluid. Stage II: The continuing filtration of liquid and solutes overpowers the pumping capacity of the lymphatic system. The fluid initially collects in the more compliant interstitial compartment. Stage III: As fluid filtration continues to increase and the filling of loose interstitial space occurs, fluid accumulation in the less compliant compartment takes place. In certain cases, the interstitial space may contain up to 500 ml of fluid. Eventually, if the accumulation continues, the fluid may cross the alveolar epithelium in to the alveoli, leading to alveolar flooding. Hence, incipient pulmonary edema is an effective indicator of worsening CHF.

Furthermore, many heart diseases can also be identified by detecting changes of certain variables or parameters indicative of different functions of the heart, such as the systolic and diastolic slopes, pre-ejection period and left ventricular ejection time.

Electrical bio-impedance signals has been found to be an effective measure for identifying changes of many different conditions in the body of a patient, such as incipient pulmonary edema and the progression of pulmonary edema due to CHF. For example, the accumulation of fluids in the lung-region associated with pulmonary edema affects the thoracic impedance, or more specifically the DC impedance level, since the resistivity of the lung changes in accordance with a change of the ratio of fluid to air. The DC impedance level is negatively correlated with the amount of fluids in the lung. Studies have shown that hospitalization due to the development of acute CHF with the symptom pulmonary edema was preceded two or three weeks by a drop in the DC impedance by approximately 10-15 %.

In addition to the thoracic impedance, the cardiogenic impedance, which is defined as the impedance or resistance variation that origins from cardiac contractions measured by electrodes inside or on the surface of the body, can be used for identifying changes of different conditions in the heart of a patient. For example, parameters such as the systolic and diastolic slopes, pre-ejection period and left ventricular ejection time indicative of different functions of the heart can be extracted from the cardiogenic impedance. The impedance is calculated as z=u/i, where u is the measured voltage between two electrodes and i is the applied excitation current between the two electrodes. The electrodes are placed inside or on the surface of the heart, integrated on a pacemaker lead or outside of the heart such as the pacemaker encapsulation. The cardiogenic impedance variation correlates to the volume changes of the heart chambers, which can be used as an indication of the dynamic blood filling. Hence, changes of these parameters due to a change in the heart, for example, caused by a disease such as heart failure can be detected by monitoring or detecting changes of the cardiogenic impedance. Several different impedance measurement configurations are known. In the most basic configuration the measurement current is injected between two electrodes and the voltage is measured between the same electrodes. The impedance is calculated as u/i. Since the impedance value is significantly affected by the tissue resistivity near the current injecting electrodes other impedance measurement configurations have been developed. The tripolar configuration uses one current injecting electrode and one voltage measurement electrode and one common electrode used for both current injection and voltage measurement. One example of such an arrangement is a configuration where the measurement current is injected between a pacemaker encapsulation and a pacing electrode tip while the voltage is measured between the pacing electrode ring or indifferent electrode and the pacemaker encapsulation. This configuration has the advantage that it improves the measurement sensitivity for tissue resistivity variations for tissue located at some distance from the electrodes used for impedance measurement. This configuration, referred to as tripolar configuration, improves the sensitivity for pulmonary edema monitoring. In a further improvement two separate electrodes are used for current injection and two separate electrodes are used for voltage measurement. This last configuration is commonly referred to as quadropolar configuration.

Accordingly, an effective method for measuring or detecting changes in electrical bio-impedances, such as the intra thoracic impedance or the cardiogenic impedance, i.e. the cardiac component of an impedance signal measured over the heart, would be of a great value. However, a problem associated with such measuring methods is the accurateness and reliability of the obtained signals since they are greatly affected by factors like the body position of the patient, patient activity levels, heart rate frequency, etc.. For example, it has been found that the body position of the patient is of major importance with regard to the thoracic impedance as well as the cardiogenic impedance. Moreover, it has also been found that the heart rate frequency has a major impact on the cardiogenic impedance. A number of attempts to eliminate or filter out these error sources have therefore been proposed. For example, U.S. Pat. No. 6,104,949 discloses a method and device for treatment of CHF, in which changes in the posture of the patient is correlated with changes of the trans-thoracic impedance. A posture sensing means indicates whether the patient lies down or is standing and the measurement of the trans-thoracic impedance is then correlated with periods when the patient is lying down or standing up.

However, is has recently been found that the position dependence also is of a significant magnitude regarding different positions even when the patient is lying down, for example, whether the patient is lying on a side or is lying on the back. A major reason is that an impedance measurement depends on the measurement vector, i.e. the vector between the nodes that the current is applied between and the vector the voltage is measured between. When the body shifts position, these vectors will change since the gravity will influence, for example, tissue between the nodes and how it moves. Tests performed on animals have shown that the trans thoracic impedance may vary up to 20 % depending on which position the animal was lying in.

US 2004/0102712 describes techniques for measuring electrical impedance across lung tissue so that pulmonary edema or its onset may be detected. More specifically, an implantable device including a lung edema impedance circuit that measures the impedance between a lead electrode and a can electrode is described. The DC component of the obtained impedance is used to determine a degree of pulmonary edema. In addition, the device may include a posture sensor providing patient orientation information. Based on posture sensor information, the relationship between the impedance measurement and the degree of edema may be adjusted to compensate for the orientation of the patient. Furthermore, in one implementation of the invention of US 2004/0102712, edema may be assessed at about the same heart rates and postures. One way this could be accomplished is by executing the steps for edema assessments only when the heart rate and posture fall within pre-programmed ranges.

Accordingly, there is a need of an improved medical device that is able to obtain electrical bio-impedance signals in order to monitor or detect changes of a condition of a patient in a more reliable and accurate manner.

### BRIEF DESCRIPTION OF THE INVENTION

Thus, an object of the present invention is to provide an improved medical device that is able to obtain electrical bio-impedance signals in order to monitor or detect changes of a condition of a patient in a more reliable and accurate manner

This and other objects are achieved according to the present invention by providing medical devices having the features defmed in the independent claim. Preferable embodiments of the invention are characterised by the dependent claims.

The measured impedance consists of a DC component and an AC component, where the DC component is the baseline around which the AC component fluctuates. The DC component reflects the amount of tissue and fluids that are located between the measuring points that the impedance is measured in-between and the AC components reflects how respiration and cardiac activity influence the impedance signal.

For the purpose of clarity, the term "intra thoracic impedance" refers to an impedance measurement over the thorax by using an implantable medical device, i.e. an impedance measurement where the impedance measurement vector spans over the thorax.

Moreover, in order to clarify, the term "cardiogenic impedance" is defined as the impedance or resistance variation that origins from cardiac contractions or, in other words, the cardiac component of the impedance measured between electrodes within the heart.

Thus, the invention is based on the idea of measuring the electrical bio-impedance only when the patient is in a predetermined specific body position. By performing the impedance measurement only in this specific position the impedance signals that are substantially repeatable can be obtained. Thereby, changes of a condition of the patient or trends in the development of a condition of a patient can be monitored or detected in an effective way.

This solution provides several advantages over the existing solutions. One advantage is that the obtained signals are very accurate and reliable since the measurements are performed only when the patient is in a predetermined specific body position. This entails that variations in the signals due to measurements in different body positions can be substantially eliminated, which is an evident risk with the method disclosed in U.S. Pat. No. 6,104,949 where the impedance measurements is correlated with moments when the patient is lying down and, therefore, the measurements are, in practical, performed in a number of different positions, i.e. when the patient is lying on either side or when the patient is lying on the back, etc.

Another advantage is that the measurements are initiated only when the patient is in the specific predetermined position whereby a more efficient method with respect to current consumption is achieved in comparison with the method according to U.S. Pat. No. 6,104,949 where the impedance measurements are performed on a constant basis and when it is detected that the patient is lying down the measurement values for the assessing of the degree of heart failure are obtained and stored.

In accordance with one embodiment of the present invention, the specific body position when the patient is lying on the back.

The cardiac component of the electrical bio-impedance is sensed, which can be used for identifying changes different conditions in the heart of a patient.

The cardiac component of the electrical bio-impedance can be used to extract surrogates of the heart function from the group of: systolic and diastolic slopes, the pre-ejection period, or the left ventricular ejection time.

Further objects and advantages of the present invention will be discussed below by means of exemplifying embodiments.

### BRIEF DESCRIPTION OF THE DRAWINGS

In the following detailed description, reference will be made to the accompanying drawings, of which:
Fig. 1 is schematic diagram showing a medical device implanted in a patient in which device the present invention can be implemented.
Fig. 2 is block diagram of the primary functional components of a first embodiment of the present invention.
Figs. 3a, 3b, and 3c are schematic diagrams of a first embodiment of the position detecting sensor of Fig. 1.
Fig. 4 is a flow chart illustrating the steps in accordance with one embodiment of the present invention to measure the electrical bio-impedance indicative of changes of a condition of the patient or trends in the development of a condition of a patient.

### DETAILED DESCRIPTION OF THE INVENTION

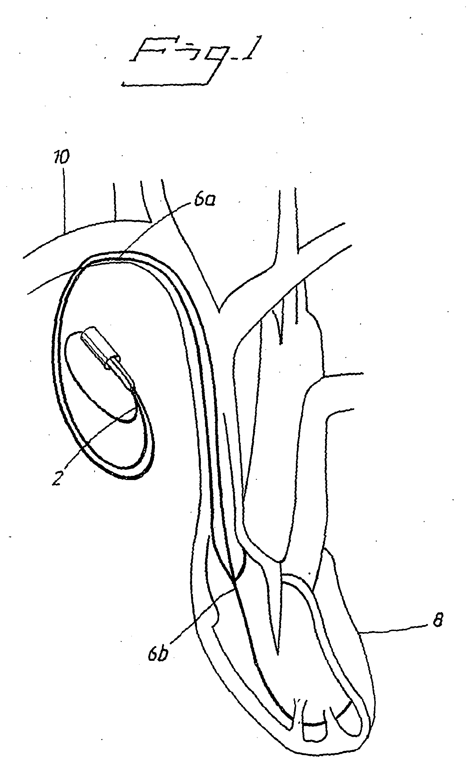

With reference to Fig. 1 there is shown a schematic diagram of a medical device implanted in a patient in which device the present invention can be implemented. As seen, this embodiment of the present invention is shown in the context of a pacemaker 2 implanted in a patient (not shown). The pacemaker 2 comprises a housing being hermetically sealed and biological inert. Normally, the housing is conductive and may, thus, serve as an electrode. One or more pacemaker leads, where only two are shown in Fig. 1 namely a ventricular lead 6a and an atrial lead 6b, are electrically coupled to the pacemaker 2 in a conventional manner. The leads 6a, 6b extend into the heart 8 via a vein 10 of the patient. One or more conductive electrodes for receiving electrical cardiac signals and/or for delivering electrical pacing to the heart 8 are arranged near the distal ends of the leads 6a, 6b. As the skilled man in the art realizes, the leads 6a, 6b may be implanted with its distal end located in either the atrium or ventricle of the heart 8.

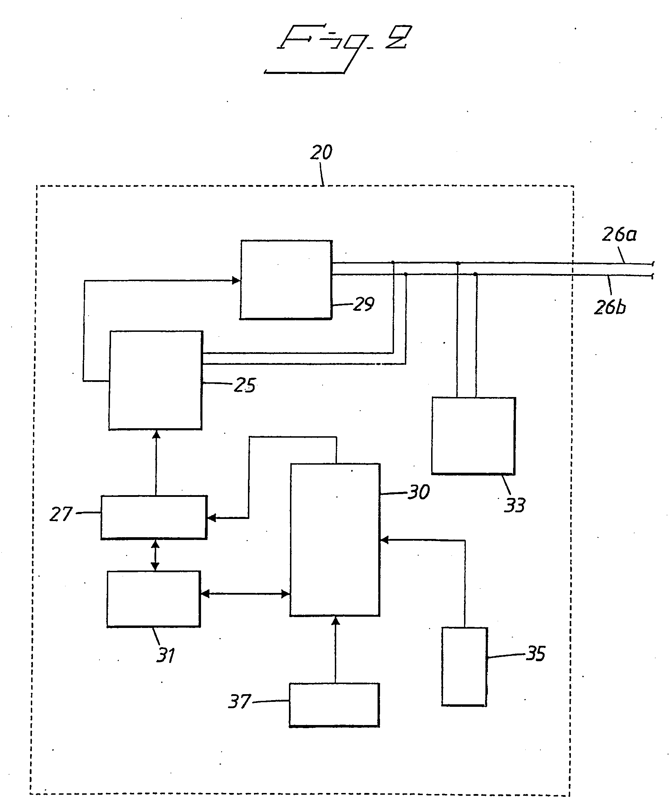

With reference now to Fig. 2, the configuration including the primary components of an embodiment of the present invention will be described. The illustrated embodiment comprises an implantable medical device 20, such as the pacemaker shown in Fig. 1, and leads 26a and 26b, of the same type as the leads 6a and 6b shown in Fig. 1, for delivering signals between the implantable medical device 20. The leads 26a, 26b may be unipolar or bipolar, and may include any of the passive or active fixation means known in the art for fixation of the lead to the cardiac tissue. As an example, the lead distal tip (not shown) may include a tined tip or a fixation helix. The leads 26a, 26b comprises one or more electrodes (as described with reference to fig. 1), such as a tip electrode or a ring electrode, arranged to, inter alia, transmit pacing pulses for causing depolarization of cardiac tissue adjacent to the electrode(-s) generated by a pace pulse generator 25 under influence of a control circuit 27. The control circuit 27 controls pace pulse parameters such as output voltage and pulse duration. Moreover, an impedance circuit 29 is arranged to carry out the impedance measurements. The impedance circuit is arranged to apply excitation current pulses between any of the implanted electrodes. The electrodes used for impedance measurement may be, for example, unipolar or bipolar electrodes located in or on the right atrium, the left atruim, the right ventricle or the left ventricle. Further the pacemaker encapsulation is frequently used as an electrode for impedance measurements. The voltage measurements made by the impedance circuit may be between the electrodes used for current injection or between other electrodes. The electrodes used for impedance measurement are selected depending on the purpose of the impedance measurement. For for intrathoracic measurements such as pulmonary edema monitoring it is essential to include tissue outside of the heart in the impedance measurement and in this case at least one electrode outside of the heart such as the pacemaker encapsulation should be used in the impedance measurement configuration. For monitoring of the heart such as cardiak stroke volume, diastolic and systolic slope etc.at least one electrode should be located inside the heart in the impedance measurement circuit. Further, the impedance circuit 29 is coupled to a microprocessor 30, where processing of the obtained impedance signals is performed. In an embodiment where the cardiac component of the electrical bio-impedance is sensed, the impedance circuit 29 is arranged to apply an excitation current pulse between a first electrode and a second electrode arranged to be positioned at different position within the heart of the patient and to sense the impedance in the tissues between the first and second electrode to the excitation current pulse. The microprocessor 30 may be arranged to extract the cardiac component of the sensed impedance. This cardiac component can be used for calculating parameters like systolic and diastolic slopes, the pre-ejection period, or left ventricular ejection time. This calculation can be performed in accordance with conventional practice within the art.

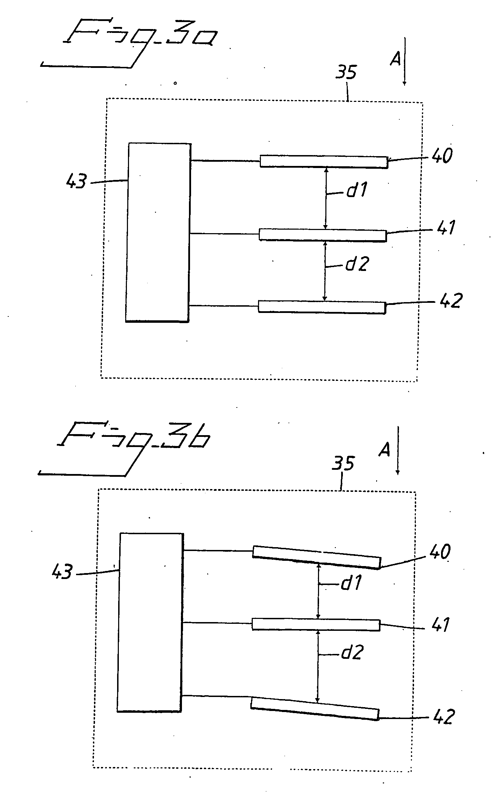

The impedance measuring circuit 29 is controlled by the microprocessor 30 and the control circuit 27. The control circuit 27 acts under influence of the microprocessor 30. A storage means 31 is connected to the control circuit 27 and the microprocessor 30, which storage means 31 may include a random access memory (RAM) and/or a non-volatile memory such as a read-only memory (ROM). Detected signals from the patients heart are processed in an input circuit 33 and are forwarded to the microprocessor 30 for use in logic timing determination in known manner. Furthermore, the implantable medical device 20 according to the present invention comprises position detecting sensor 35 arranged to detect a predetermined, specific body position of said patient. In a preferred embodiment of the present invention, the position detecting means is a back-position sensor arranged to sense when the patient is lying on his/hers back (or on his or hers face), see, for example, Fig. 3a. The position detecting sensor 35 is connected to the microprocessor 30. The implantable medical device 20 is powered by a battery 37, which supplies electrical power to all electrical active components of the medical device 20. Data contained in the storage means 31 can be transferred to a programmer (not shown) via a programmer interface (not shown) for use in analyzing system conditions, patient information, calculation of surrogate parameters such as systolic and diastolic slopes, the pre-ejection period, or left ventricular ejection time and changing pacing conditions.

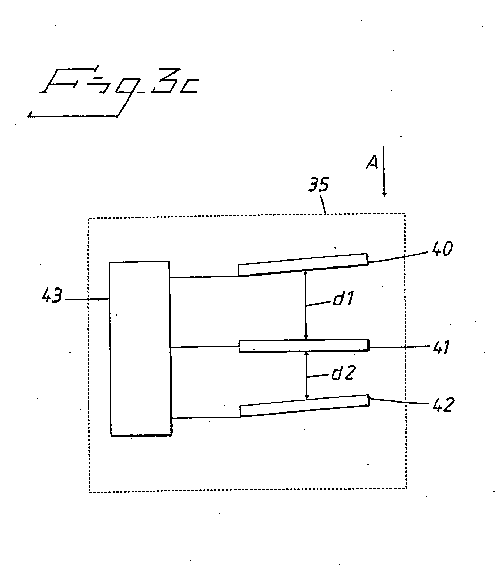

With reference now to Fig. 3a, 3b and 3c, a preferred embodiment of the position detecting sensor will be described. According to this embodiment, the position detecting sensor 35 comprises a first conducting plate 40, a second conducting plate 41, and a third conducting plate 42, wherein the first and second plate 40 and 41, respectively are spaced apart with a first distance d₁ and the second and third plate 41 and 42, respectively, are spaced apart with a second distance d₂, see Fig. 3a. Each plate 40, 41, 42 is connected to a discriminating circuit 43 arranged to sense a first capacitance c₁ between the first and second plate 40 and 41, respectively, and a second capacitance c₂ between the second and third plate 41 and 42, respectively. According to one embodiment, the first and second capacitor plate 40 and 42 are flexible. In another embodiment, the first and second capacitor plate 40 and 42 are pivotally suspended. Preferably, the first and second capacitor plate 40 and 42 are arranged to, when the sensor is positioned such that the plates 40-42 are substantially parallel with ground, will move, i.e. bend or pivot, slightly against the ground under the influence of gravity. Thereby, the first and second distance d₁ and d₂ will change and there will, in turn, arise a difference between the first capacitance c₁ and c₂, which can be sensed by the discriminating circuit 43. When the first distance d₁ is shorter than the second distance d₂, the first capacitance c₁ will be larger than the second capacitance c₂, see Fig. 3b. In this case the sensor is arranged to deliver a positive signal, c₁-c₂. Inversely, when the first distance d₁ is longer than the second distance d₂, the first capacitance c₁ will be smaller than the second capacitance c₂, see Fig. 3c. Accordingly, the sensor will deliver a negative signal c₁-c₂.

Moreover, in this embodiment, the first and second distance d₁ and d₂ are equal and the plates 40-42 are arranged so that the first capacitance c₁ is equal to the second capacitance ₂ when the sensor is positioned such that the capacitor plates 40-42 are perpendicular or forming an angle with respect to the ground. Consequently, when the patient is in positions such that the capacitor plates 40-42 are perpendicular or forming an angle with respect to the ground, the sensor 35 will not deliver any signal since c₁ is equal to c₂.

Preferably, the sensor is installed in an implantable medical device such that there will arise a difference between c₁ and c₂ when the patient carrying the device lies on his or hers back (or on his or hers face), due to the fact that plates 40 and 42 are positioned substantially parallel to the ground and therefore will move, i.e. bend or pivot, against ground, and such that the plates 40 and 42 are not affected by the gravity when the patient is in other positions, for example, lying on his or hers side or standing. For example, when the patient is lying on his or hers back, the sensor is arranged such that the first plate 40 and the second plate 42 will bend in the direction indicated by the arrow A, thereby the first distance d₁ will be shorter than the second distance d₂ and the first capacitance c₁ will be larger than the second capacitance c₂, see Fig. 3b. In this case, the sensor is arranged to deliver a positive signal, c₁-c₂. Inversely, when the patient is lying on the face, the first plate 40 and the second plate 42 will bend in a direction against the arrow A, the first distance d₁ will be longer than the second distance d₂ and the first capacitance c₁ will be smaller than the second capacitance c₂, see Fig. 3c. Accordingly, the sensor will deliver a negative signal c₁-c₂. Thus, the position sensor 35 is capable of discriminating between different horizontal positions of the patient.

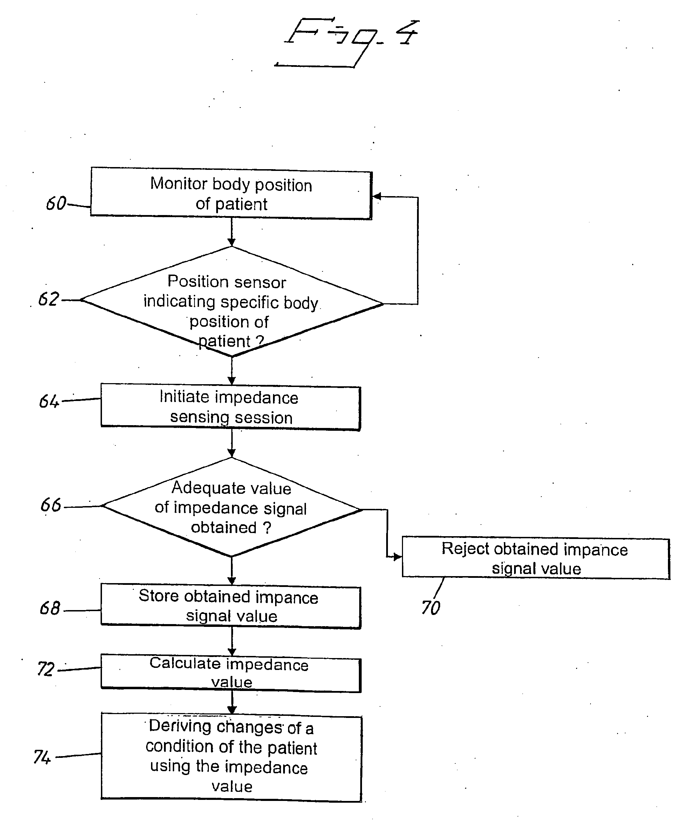

Referring now to Fig. 4, a high-level description of the method according to the present invention will be given. At step 60, the position sensor 35 monitors or detects the position of the patient in order to detect a predetermined specific body position of the patient, i.e. the sensor is arranged to supply a position indicating signal when the patient is in the specific position as described above. In a preferred embodiment, the specific predetermined body position is when the patient is lying on the back (or on the face). During periods when the patient is in other positions than the predetermined specific position, the impedance measuring circuit 29 is in an idle mode. When the patient is the specific body position, the sensor, in step 62, supplies a position indicating signal or triggering signal to the microprocessor 30. The microprocessor influences the control circuit 27, which, in turn, puts the impedance measuring circuit 29 in an active mode where the measuring circuit 29 initiates an impedance measuring session, which will be described below. Thereafter, at step 66, it is judged whether the obtained impedance signals value is valid. This can be performed, for example, by checking that the obtained value is within a preset range including the preceding value. If the obtained impedance signal is found to be valid, it is stored temporarily, at step 68, in the storage means 31. If the obtained value is found to be invalid, i.e. the value being outside the preset range, the signal is rejected. In one embodiment, an new impedance measuring session is initiated after a delay period of a predetermined length and if this is repeated a preset number of times without obtaining a valid signal, the impedance measuring circuit returns to the idle mode. At step 72, the stored impedance signals is used to calculate impedance values. This calculation can be performed through execution of suitable software in the microprocessor 30. Thereafter, at step 74, the calculated values is compared with stored impedance values obtained in earlier impedance measuring sessions in order to monitor, for example, changes and/or trends of the development of the impedance. Thereby, it can be derived whether a condition of the patient influencing the impedance is changing, for example, congestive heart failure.

As mentioned above, electrical bio-impedance signals has been found to constitute an effective measure for identifying changes of many different conditions in the body of a patient. According to a preferred embodiment, the obtained impedance signals are utilized to monitor or detect incipient pulmonary edema and the progression of pulmonary edema due to CHF. Since the accumulation of fluids in the lung-region associated with pulmonary edema affects the thoracic impedance, or more specifically the DC impedance level, due to the fact that the resistivity of the lung changes in accordance with a change of the ratio of fluid to air, trends and/or changes of the impedance levels constitute a useful measure in order to monitor or detect incipient edema. The DC impedance level is negatively correlated with the amount of fluids in the lung. There are a number of possible impedance configurations, i.e. ways of injecting current between two electrodes in the pacemaker and then to measure the voltage the current provokes between the electrodes. For example, impedance configurations can be uni-polar, bi-polar, tri-polar or quadro-polar. The configuration denominated as bi-polar means, in practice, a configuration where the current and the voltage is sent out and measured between the same two electrodes. When one of the electrodes used in a bi-polar measurement is the housing or the case, the configuration is called uni-polar. For example, in Fig. 1, between the housing of the pacemaker 2 and a right ventricular electrode arranged at the distal end of lead 6a. A tri-polar configuration uses three electrodes, i.e. the current injection and the voltage measurement share one electrode. As an example, the current can be sent out from the housing or the case of the medical device to a RV-tip and the voltage is measured between the case and RV-ring. In quadro-polar measurements, the current is sent out between electrodes and the voltage is measured between two entirely different electrodes, i.e. in this case there are four electrodes involved.

According to embodiments of the present invention, different measurements conditions can be specified in order to obtain more accurate impedance values. For example, the initiation of the impedance measuring session can be delayed a predetermined period of time, for example 0-10h, after that the signal indicative that the patient is in the specific position. Furthermore, according to another embodiment of the present invention, a condition for initiating the impedance measuring session is that a sensed activity level of the patient is within a predetermined range. The activity level can be sensed be means of an activity sensor incorporated in the medical device in accordance with conventional practice within the art. That is, even if the patient is in the specific position, the impedance measuring session is initiated only if the activity level signal is within the predetermined range.

As mentioned above, also electrical bio-impedance signals has been found to constitute an effective measure for identifying changes of many different conditions in the body of a patient, and according to one embodiment of the present invention, the cardiac component of the impedance measured between electrodes within the heart is used to calculate surrogates for heart failure. Thus, by monitoring or detecting trends and/or changes of these surrogates, for example, parameters, such as the systolic and diastolic slopes, pre-ejection period and left ventricular ejection time, progress of conditions such as CHF can be studied. The cardiogenic impedance is defined as the impedance or resistance variation that origins from cardiac contractions measured by electrodes inside or on the surface of the body. The impedance is calculated as z=u/i, where u is the measured voltage between two electrodes and i is the applied excitation current between the two electrodes. Normally, the electrodes are placed inside or on the surface of the heart, integrated on a pacemaker lead, for example the leads 6a, 6b shown in Fig. 1. The cardiogenic impedance variation correlates to the volume changes of the heart chambers, which can be used as an indication of the dynamic blood filling. Preferably, the microprocessor 30 is arranged to filter the cardiac component from the obtained electrical bio-impedance and to extract systolic and diastolic slopes, the pre-ejection period, or left ventricular ejection time using the data corresponding to the cardiac component of the bio-impedance signals obtained in the impedance measuring session. In addition, this extracting procedure can be performed in an external unit, wherein the filtered cardiac component is transferred from the medical device via the telemetry device (not shown).

According to embodiments of the present invention, different measurements conditions can be specified in order to obtain more accurate impedance values. As an example, the impedance measurements can be correlated with the heart rate of the patient. For this purpose, the heart rate of the patient is sensed and it is determined whether the sensed heart rate is within a predetermined range, and the impedance measuring session is initiated only if the heart rate is within the predetermined range. That is, even if the patient is in the specific position the impedance measuring session is initiated only if the heart rate is within the predetermined range. In this embodiment, means for sensing the heart rate of the patient is incorporated in the medical deice in accordance with conventional practice within the art.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. Medical device (20) for detecting a change of a condition of a patient, comprising:
an impedance measurement circuit (29) for measuring an electrical bio-impedance associated with said condition, and
a position sensor (35) for detecting a body position of said patient, whereby said position sensor (35) is arranged to detect a predetermined, specific body position of said patient and, when detecting that the patient is in said specific body position, to apply a triggering signal to said impedance measuring circuit (29), and whereby said impedance measurement circuit (29) is arranged to, upon receiving said triggering signal, initiate an impedance measuring session in order to obtain electrical bio-impedance signals when said patient is in said predetermined, specific body position,
**characterized in**
**that** said medical device (20) further comprises a microprocessor (30) which is arranged to extract a cardiac component of said obtained electrical bio-impedance signals and to calculate a parameter to be monitored for detection of a change of said condition, whereby said microprocessor (30) is arranged to calculate said parameter from said cardiac component of said obtained electrical bio-impedance signals.

2. Medical device (20) according to claim 1, wherein said position sensor is arranged to detect when said patient is lying on the back and to apply said triggering signal when said patient is lying on the back.

3. Medical device (20) according to claim 1 or 2, further comprising a memory (31) for storing impedance data corresponding to said electrical bio-impedance signals obtained in said impedance measuring session.

4. Medical device (20) according to any one of claims 1-3, wherein said impedance measurement circuit (29) comprises:
a first electrode and a second electrode of which at least one electrode is arranged to be positioned within a heart (8) of the patient;
means for applying an excitation current pulse between the first and second electrode, and
means for measuring the impedance in the tissues between the first and second electrode to the excitation current pulse.

5. Medical device (20) according to claim 1 or 2, further comprising a measurement circuit for measuring a heart rate of the patient and a heart rate comparing circuit for detecting when the heart rate is within a predetermined range, wherein said heart rate comparing circuit is arranged to apply a heart rate indicating signal to said impedance measuring circuit (29) when the heart rate is within a predetermined range thereby initiating an impedance measuring session.

6. Medical device (20) according to any one of claims 1-5, wherein said position sensor (35) is arranged to, when the specific body position is sensed, apply said triggering signal to a control circuit (27); and
wherein said control circuit (27), upon receiving said triggering signal, is adapted to bring said impedance measurement circuit (29) to delay the initiating of the impedance measuring session a predetermined period of time.

7. Medical device (20) according to any one of claims 1-6, further comprising activity measuring means arranged to measure an activity level of said patient, wherein said activity measuring means is arranged to apply an activity level signal to a control circuit (27), and
said control circuit (27) is adapted to determine whether said activity level signal is within a predetermined range, and, if said activity level signal is within the predetermined range, bring said impedance measuring circuit (29) to initiate said impedance measuring session.

8. Medical device (20) according to any one of claims 1-4, wherein said microprocessor (30) is arranged to calculate a systolic slope and a diastolic slope using data corresponding to the cardiac component of the impedance signals obtained in said impedance measuring session.

9. Medical device (20) according to any one of claims 1-4, wherein said microprocessor (30) is arranged to calculate a pre-ejection period using data corresponding to the cardiac component of the bio-impedance signals obtained in said impedance measuring session.

10. Medical device (20) according to any one of claims 1-4, wherein said microprocessor (30) is arranged to calculate a left ventricular ejection time using data corresponding to the cardiac component of the bio-impedance signals obtained in said impedance measuring session.

## Patentansprüche

1. Medizinische Vorrichtung (20) zum Erfassen einer Veränderung eines Zustands eines Patienten, umfassend:
eine Impedanzmessschaltung (29) zum Messen einer elektrischen Bioimpedanz, die dem Zustand zugehörig ist, und
einen Positionssensor (35) zum Erfassen einer Körperposition des Patienten, wodurch der Positionssensor (35) angeordnet ist, eine vorher festgelegte bestimmte Körperposition des Patienten zu erfassen und, wenn er erfasst, dass sich der Patient in der bestimmten Körperposition befindet, ein Auslösesignal an die Impedanzmessschaltung (29) anzulegen, und wodurch die Impedanzmessschaltung (29) angeordnet ist, beim Empfangen des Auslösesignals einen Impedanzmessvorgang zu starten, um elektrische Bioimpedanzsignale zu erhalten, wenn sich der Patient in der vorher festgelegten bestimmten Körperposition befindet,
**dadurch gekennzeichnet,**
**dass** die medizinische Vorrichtung (20) ferner einen Mikroprozessor (30) umfasst, der angeordnet ist, aus den erhaltenen elektrischen Bioimpedanzsignalen eine Herzkomponente zu entnehmen und einen Parameter zu berechnen, der zum Erfassen einer Veränderung des Zustands überwacht werden soll, wodurch der Mikroprozessor (30) angeordnet ist, den Parameter aus der Herzkomponente der erhaltenen elektrischen Bioimpedanzsignale zu berechnen.

2. Medizinische Vorrichtung (20) nach Anspruch 1, wobei der Positionssensor angeordnet ist, um zu erfassen, wann der Patient auf dem Rücken liegt, und das Auslösesignal anzulegen, wenn der Patient auf dem Rücken liegt.

3. Medizinische Vorrichtung (20) nach Anspruch 1 oder 2, ferner umfassend einen Speicher (31) zum Speichern von Impedanzdaten, die den elektrischen Bioimpedanzsignalen entsprechen, die bei dem Impedanzmessvorgang erhalten werden.

4. Medizinische Vorrichtung (20) nach einem der Ansprüche 1 bis 3, wobei die Impedanzmessschaltung (29) Folgendes umfasst:
eine erste Elektrode und eine zweite Elektrode, von denen mindestens eine Elektrode angeordnet ist, im Herz (8) des Patienten platziert zu werden;
Mittel zum Anlegen eines Anregungsstromimpulses zwischen der ersten und zweiten Elektrode, und
Mittel zum Messen der Impedanz gegenüber dem Anregungsstromimpuls in dem Gewebe zwischen der ersten und zweiten Elektrode.

5. Medizinische Vorrichtung (20) nach Anspruch 1 oder 2, ferner umfassend eine Messschaltung zum Messen einer Herzfrequenz des Patienten und eine Herzfrequenzvergleichsschaltung zum Erfassen, wann die Herzfrequenz in einem vorher festgelegten Bereich liegt, wobei die Herzfrequenzvergleichsschaltung angeordnet ist, ein Herzfrequenzhinweissignal an die Impedanzmessschaltung (29) anzulegen, wenn die Herzfrequenz in einem bestimmten Bereich liegt, wodurch ein Impedanzmessvorgang gestartet wird.

6. Medizinische Vorrichtung (20) nach einem der Ansprüche 1 bis 5, wobei der Positionssensor (35) angeordnet ist, das Auslösesignal an eine Steuerschaltung (27) anzulegen, wenn die bestimmte Körperposition erfasst wird; und
wobei die Steuerschaltung (27) beim Empfangen des Auslösesignals angepasst ist, die Impedanzmessschaltung (29) dazu zu veranlassen, den Start des Impedanzmessvorgangs um einen festgelegten Zeitraum zu verschieben.

7. Medizinische Vorrichtung (20) nach einem der Ansprüche 1 bis 6, ferner umfassend ein Aktivitätsmessmittel, das angeordnet ist, einen Aktivitätsgrad des Patienten zu messen, wobei das Aktivitätsmessmittel angeordnet ist, ein Aktivitätsgradsignal an eine Steuerschaltung (27) anzulegen, und
die Steuerschaltung (27) angepasst ist, zu ermitteln, ob das Aktivitätsgradsignal in einem bestimmten Bereich liegt, und wenn das Aktivitätsgradsignal in dem bestimmten Bereich liegt, die Impedanzmessschaltung (29) dazu zu veranlassen, den Impedanzmessvorgang zu starten.

8. Medizinische Vorrichtung (20) nach einem der Ansprüche 1 bis 4, wobei der Mikroprozessor (30) angeordnet ist, eine systolische Steigung und eine diastolische Steigung unter Verwendung von Daten zu berechnen, die der Herzkomponente der Impedanzsignale entsprechen, die bei dem Impedanzmessvorgang erhalten werden.

9. Medizinische Vorrichtung (20) nach einem der Ansprüche 1 bis 4, wobei der Mikroprozessor (30) angeordnet ist, einen Zeitraum vor der Austreibung unter Verwendung von Daten zu berechnen, die der Herzkomponente der Bioimpedanzsignale entsprechen, die bei dem Impedanzmessvorgang erhalten werden.

10. Medizinische Vorrichtung (20) nach einem der Ansprüche 1 bis 4, wobei der Mikroprozessor (30) angeordnet ist, eine linksventrikuläre Austreibungszeit unter Verwendung von Daten zu berechnen, die der Herzkomponente der Bioimpedanzsignale entsprechen, die bei dem Impedanzmessvorgang erhalten werden.

## Revendications

1. Dispositif médical (20) pour détecter un changement d'état d'un patient, comprenant :
un circuit de mesure d'impédance (29) pour mesurer une bioimpédance électrique associée audit état, et
un capteur de position (35) pour détecter une position corporelle dudit patient, ce par quoi ledit capteur de position (35) est disposé pour détecter une position corporelle spécifique prédéterminée dudit patient et, quand il détecte que le patient est dans ladite position corporelle spécifique, pour appliquer un signal de déclenchement audit circuit de mesure d'impédance (29), et ce par quoi ledit circuit de mesure d'impédance (29) est disposé pour, lors de la réception dudit signal de déclenchement, lancer une session de mesure d'impédance afin d'obtenir des signaux de bioimpédance électrique quand ledit patient est dans ladite position corporelle spécifique prédéterminée,
**caractérisé en ce**
**que** ledit dispositif médical (20) comprend en outre un microprocesseur (30) qui est disposé pour extraire une composante cardiaque desdits signaux de bioimpédance électrique obtenus et pour calculer un paramètre à surveiller pour la détection d'un changement dudit état, ce par quoi ledit microprocesseur (30) étant disposé pour calculer ledit paramètre à partir de ladite composante cardiaque desdits signaux de bioimpédance électrique obtenus.

2. Dispositif médical (20) selon la revendication 1, dans lequel ledit capteur de position est disposé pour détecter quand ledit patient est allongé sur le dos et pour appliquer ledit signal de déclenchement quand ledit patient est allongé sur le dos.

3. Dispositif médical (20) selon la revendication 1 ou 2, comprenant en outre une mémoire (31) pour stocker des données d'impédance correspondant auxdits signaux de bioimpédance électrique obtenus dans ladite session de mesure d'impédance.

4. Dispositif médical (20) selon l'une quelconque des revendications 1 à 3, dans lequel ledit circuit de mesure d'impédance (29) comprend :
une première électrode et une seconde électrode desquelles au moins une électrode est disposée pour être positionnée à l'intérieur d'un coeur (8) du patient ;
des moyens pour appliquer une impulsion de courant d'excitation entre la première et la seconde électrodes, et
des moyens pour mesurer l'impédance à l'impulsion de courant d'excitation dans les tissus entre la première et la seconde électrodes.

5. Dispositif médical (20) selon la revendication 1 ou 2, comprenant en outre un circuit de mesure pour mesurer un rythme cardiaque du patient et un circuit de comparaison de rythme cardiaque pour détecter quand le rythme cardiaque est dans un intervalle prédéterminé, dans lequel ledit circuit de comparaison de rythme cardiaque est disposé pour appliquer un signal d'indication de rythme cardiaque audit circuit de mesure d'impédance (29) quand le rythme cardiaque est dans un intervalle prédéterminé, lançant ainsi une session de mesure d'impédance.

6. Dispositif médical (20) selon l'une quelconque des revendications 1 à 5, dans lequel ledit capteur de position (35) est disposé pour, quand la position corporelle spécifique est détectée, appliquer ledit signal de déclenchement à un circuit de contrôle (27) ; et
dans lequel ledit circuit de contrôle (27), lorsqu'il reçoit ledit signal de déclenchement, est adapté pour amener ledit circuit de mesure d'impédance (29) à retarder le lancement de la session de mesure d'impédance d'une période de temps prédéterminée.

7. Dispositif médical (20) selon l'une quelconque des revendications 1 à 6, comprenant en outre un moyen de mesure d'activité disposé pour mesurer un niveau d'activité dudit patient, dans lequel ledit moyen de mesure d'activité est disposé pour appliquer un signal de niveau d'activité à un circuit de contrôle (27), et
ledit circuit de contrôle (27) est adapté pour déterminer si ledit signal de niveau d'activité est dans un intervalle prédéterminé, et, si ledit signal de niveau d'activité est dans l'intervalle prédéterminé, amener ledit circuit de mesure d'impédance (29) à lancer ladite session de mesure d'impédance.

8. Dispositif médical (20) selon l'une quelconque des revendications 1 à 4, dans lequel ledit microprocesseur (30) est disposé pour calculer une pente systolique et une pente diastolique en utilisant des données correspondant à la composante cardiaque des signaux d'impédance obtenus dans ladite session de mesure d'impédance.

9. Dispositif médical (20) selon l'une quelconque des revendications 1 à 4, dans lequel ledit microprocesseur (30) est disposé pour calculer une période de prééjection en utilisant des données correspondant à la composante cardiaque des signaux de bioimpédance obtenus dans ladite session de mesure d'impédance.

10. Dispositif médical (20) selon l'une quelconque des revendications 1 à 4, dans lequel ledit microprocesseur (30) est disposé pour calculer un temps d'éjection ventriculaire gauche en utilisant des données correspondant à la composante cardiaque des signaux de bioimpédance obtenus dans ladite session de mesure d'impédance.
